# EUROPÄISCHE PATENTANMELDUNG

(11) **EP 1 634 568 A1**
(43) Veröffentlichungstag der Anmeldung: **15.03.2006**
(21) Anmeldenummer: 05014918.6
(22) Anmeldetag: 09.07.2005
(51) Int. Cl.: A61K 8/20, A61K 8/23, A61K 8/34, A61K 8/39, A61K 8/60, A61K 8/86, A61Q 19/10, A61Q 1/14

(54) **Selbsterwärmende Hautreinigungszusammensetzung**

(30) Priorität: 09.08.2004 DE 102004038771
(71) Anmelder: Henkel Kommanditgesellschaft auf Aktien, 40589 Düsseldorf (DE)
(72) Erfinder: Cortekar, Hans-Wolfgang, 42855 Remscheid (DE); Scholz, Wolfhard, 47829 Krefeld (DE)

(57) **Zusammenfassung**

Die Erfindung betrifft eine selbsterwärmende Hautreinigungszusammensetzung mit ausgewählten Abrasivkomponenten zur gründlichen und dennoch schonenden Reinigung der Haut, die bei der Anwendung mit Wasser vermischt wird und dabei Wärme freisetzt.

## Beschreibung

Die Erfindung betrifft eine selbsterwärmende Hautreinigungszusammensetzung mit ausgewählten Abrasivkomponenten zur gründlichen und dennoch schonenden Reinigung der Haut, die bei der Anwendung mit Wasser vermischt wird und dabei Wärme freisetzt. Durch die freigesetzte Wärme kommt es zu einem sensorisch angenehmen Hauteindruck, einer Verbesserung des Reinigungseffektes, zu einer erhöhten Freisetzung der Duftstoffe und zu einer verbesserten Wirkung enthaltener hautkosmetischer Wirkstoffe.

Die Verwendung von wasserfreien Salzen mit negativer Lösungsenthalpie, die beim Lösen in Wasser Hydratationswärme freisetzen, zur Herstellung von kosmetischen Zubereitungen, die sich bei der Anwendung erwärmen, ist im Stand der Technik bereits bekannt. DE 2317140 C2 offenbart wässrige Haut- und Haarbehandlungsmittel, die kurz vor der Anwendung durch Hinzufügen von Calciumchlorid oder Magnesiumsulfat erwärmt werden. In DE 19624870 A1 und WO 97/02802 A2 sind Zahnpflegemittel beschrieben, die bei Zutritt von Wasser oder Speichel bei der Zahnreinigung Wärme freisetzen. In US 3,250,680 und WO 93/08793 A1 sind Hautreinigungsmittel beschrieben, die z. B. als Gesichtsmaske angewendet werden können und als Wärme freisetzende Komponente ein entwässertes Molekularsieb, das heißt einen Zeolith, in einem wasserfreien Träger enthalten. US 4,159,316 offenbart wasserfreie Zeolith-haltige Zahnpasten, in denen der Zeolith auch als Abrasivkomponente dient. US 5;747,004 offenbart Zahnpasten, die als Hydratationswärme freisetzendes Salz wasserfreies Natriumcarbonat enthalten, das in Wasser hydrolysiert und einen stark basischen pH-Wert einstellt (pH 9,36 -10,7). Derart hohe pH-Werte sind für die Anwendung auf der Haut, insbesondere der Gesichtshaut, weniger geeignet und für empfindliche Haut sogar ungeeignet. EP 1 186 286 A1 offenbart selbsterwärmende Hautreinigungsmittel mit Partikeln aus Natriumpolyacrylat von 1 - 80 µm Durchmesser, die sich bei der Anwendung mit Wasser nach und nach auflösen. In WO 01/12150 A1 findet sich der Hinweis auf eine selbsterwärmende Gesichtsmaske der Marke Bioré, die Zeolith (sodium silicoaluminate) und verkapselte Ultramarinpigmente enthält und frei ist von wasserlöslichen Salzen, die sich in Wasser mit negativer Lösungsenthalpie lösen. Beim Verreiben der Maskenzusammensetzung auf der Haut werden diese Pigmente durch die Zerstörung der Verkapselung freigesetzt und zeigen durch das Verfärben der Zusammensetzung das Ende des Reinigungsvorgangs an.

Diese bekannten Zubereitungen sind aber zur Anwendung als Hautreinigungsmittel, z. B. in Form flüssiger oder pastenförmiger Zubereitungen vom Typ einer Flüssigseife oder einer Waschpaste, weniger geeignet, da sie nicht genügend Schaum entwickeln und keine befriedigende Reinigung auch stärker verschmutzter Haut erzielt wird. Die im Stand der Technik bislang verwendeten Abrasivkomponenten sind entweder zu harsch für die Anwendung am Gesicht oder, wenn sie aus wasserlöslichen oder aus mechanisch instabilen Materialien bestehen, zu weich für eine effektive Reinigung.

Eine Aufgabe der vorliegenden Erfindung war es, ein Hautreinigungsmittel zu entwickeln, das sich wie eine Flüssigseife oder eine Handwaschpaste anwenden lässt. Eine weitere Aufgabe der vorliegenden Erfindung war es, ein Hautreinigungsmittel zu entwickeln, das einen voluminösen Schaum und gleichzeitig so viel Wärme entwickelt, dass ein sensorisch angenehmes Hautgefühl hervorgerufen wird. Eine weitere Aufgabe der vorliegenden Erfindung war es, ein Hautreinigungsmittel zu entwickeln, mit dem eine porentiefe, aber dennoch schonende Reinigung der Haut erreicht wird.

Gegenstand der vorliegenden Erfindung ist eine wasserfreie, selbsterwärmende Hautreinigungszusammensetzung, die beim Vermischen mit Wasser Hydratationswärme erzeugt, enthaltend (jeweils bezogen auf die Gesamtzusammensetzung)
a) mindestens 30 Gew.-% mindestens eines wasserlöslichen Polyols, ausgewählt aus wasserlöslichen mehrwertigen C₂ - C₉-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten,
b) mindestens 1 Gew.-% mindestens eines anionischen, zwitterionischen, ampholytischen oder nichtionischen Tensids oder Mischungen hiervon, wobei mindestens ein schäumendes Tensid enthalten ist,
c) mindestens 5 Gew.-% mindestens eines wasserlöslichen Salzes, das sich in Wasser mit negativer Lösungsenthalpie löst,
d) mindestens 0,1 Gew.-% mindestens eines organischen und/oder anorganischen Verdickungsmittels sowie
e) mindestens 0,1 Gew.-% mindestens einer organischen, wasserunlöslichen und mechanisch stabilen Abrasivkomponente mit einem mittleren Teilchendurchmesser von 50 - 400 µm.

Als wasserfrei wird dabei erfindungsgemäß eine Zusammensetzung angesehen, die maximal 2 Gew.-% Wasser, bevorzugt maximal 1 Gew.-% und besonders bevorzugt maximal 0,5 Gew.-% Wasser enthält.

Die erfindungsgemäßen Hautreinigungszusammensetzungen enthalten mindestens ein wasserlösliches Polyol, ausgewählt aus wasserlöslichen mehrwertigen C₂ - C₉-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten. Unter Wasserlöslichkeit versteht man erfindungsgemäß, dass sich wenigstens 5 Gew.-% des Polyols bei 20 °C klar lösen oder aber - im Falle langkettiger oder polymerer Polyole - durch Erwärmen der Lösung auf 50 °C bis 60 °C in Lösung gebracht werden können. In einer bevorzugten Ausführungsform der Erfindung sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose können erfindungsgemäß bevorzugt sein.

In einer weiteren bevorzugten Ausführungsform der Erfindung können einwertige C₂-C₆-Alkohole wie Ethanol, Isopropanol oder Ethoxydiglycol in einer Gesamtmenge bis 5 Gew.-% enthalten sein.

Die erfindungsgemäßen Zusammensetzungen enthalten mindestens ein wasserlösliches Polyol in einer Gesamtmenge von mindestens 30 Gew.-%. Bevorzugte Ausführungsformen der Erfindung sind dadurch gekennzeichnet, dass das/die wasserlösliche/n Polyol/e in einer Gesamtmenge von 30 - 70 Gew.-%, bevorzugt 40 - 60 Gew.-% und besonders bevorzugt 45 - 55 Gew.-%, bezogen auf die Gesamtzusammensetzung, enthalten ist/sind. Durch die Art und Menge der wasserlöslichen Polyole lässt sich die Konsistenz sowie das Löse- und Dispergierverhalten des Trägers beeinflussen. Darüber hinaus haben einige dieser wasserlöslichen Polyole ebenfalls eine negative Mischungsenthalpie mit Wasser, d. h. beim Mischen mit Wasser wird Wärme freigesetzt. Aus diesem Grund ist es besonders bevorzugt, Mischungen von zwei oder mehr unterschiedlichen wasserlöslichen Polyolen einzusetzen, um ein optimales Anwendungsprofil zu erhalten. Gemische von 1,2-Propylenglycol, Polyethylenglycol, insbesondere PEG-8, und Glycerin und gewünschtenfalls Sorbit sind außerordentlich bevorzugte Träger.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für bevorzugte anionische Tenside sind, jeweils in Form der Natrium-, Kalium- und Ammoniumsowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglycolethersulfate der Formel R-O(CH₂-CH₂O)ₓ₋OSO₃H, in der R eine bevorzugt lineare Alkylgruppe mit 8 bis 30 C-Atomen und x = 0 oder 1 - 12, bevorzugt 1 - 4, ist,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 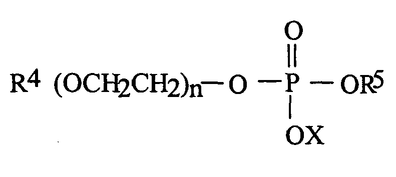 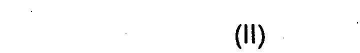 in der R⁴ bevorzugt für einen aliphatischen Kohlenwasserstoffrest mit 8 bis 30 Kohlenstoffatomen, R⁵ für Wasserstoff, einen Rest (CH₂CH₂O)ₙR¹⁸ oder X, n für Zahlen von 1 bis 10 und X für Wasserstoff, ein Alkali- oder Erdalkalimetall oder NR⁶R⁷R⁸R⁹, mit R⁶ bis R⁹ unabhängig voneinander stehend für Wasserstoff oder einen C₁ bis C₄ - Kohlenwasserstoffrest, steht,
- sulfatierte Fettsäurealkylenglycolester der Formel R¹⁰CO(AlkO)ₙSO₃M, in der R¹⁰CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV) 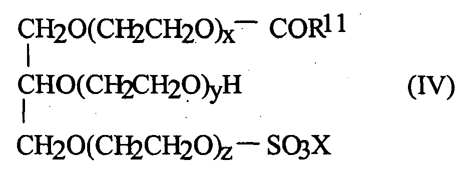 in der R¹¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht,
- Amidethercarbonsäuren gemäß EP 0 690 044,
- Kondensationsprodukte aus C₈ - C₃₀ - Fettalkoholen mit Proteinhydrolysaten und/oder Aminosäuren und deren Derivaten, sogenannte Eiweißfettsäurekondensate, z. B. Lamepon®, Gluadin®, Hostapon® KCG oder Amisoft®.

In einer bevorzugten Ausführungsform der Erfindung sind die schäumenden anionischen Tenside ausgewählt aus Alkylpolyglycolethersulfaten, Acylisethionaten, Acyltauriden, Acylsarkosiden, Sulfobernsteinsäuremonoalkylester-Salzen, Alkylsulfaten, Alkansulfonaten, Alpha-Olefinsulfonaten und Alkylpolyglycolethercarboxylaten in Form ihrer Alkali-, Magnesium-, Ammonium- oder Alkanolammoniumsalze. Besonders bevorzugt werden solche anionischen Tenside eingesetzt, die in wasserfreier, feinteiliger, das heißt pulverförmiger, Form zugänglich sind. Dies sind in der Regel die Natriumsalze der genannten Aniontenside.
Als zwitterionische Tenside werden solche oberflächenaktiven Verbindungen bezeichnet, die im Molekül mindestens eine quartäre Ammoniumgruppe und mindestens eine -COO- - oder -SO₃⁻ -Gruppe tragen. Besonders bevorzugte zwitterionische Tenside sind die sogenannten Betain-Tenside wie die N-Alkyl-N,N-dimethylammonium-glycinate, z. B. das Kokosalkyl-dimethylammoniumglycinat, N-Acyl-aminopropyl-N,N-dimethylammoniumglycinate, z. B. das Kokosacylaminopropyldimethylammoniumglycinat, das C₁₂-C₁₈-Alkyl-dimethyl-acetobetain, das Kokosamidopropyl-dimethyl-acetobetain, 2-Alkyl-3-carboxymethyl-3-hydroxyethyl-imidazoline und Sulfobetaine mit jeweils 8 bis 18 C-Atomen in der Alkyl- oder Acylgruppe sowie das Kokosacylaminoethylhydroxyethylcarboxymethylglycinat. Ein besonders bevorzugtes zwitterionisches Tensid ist das unter der INCI-Bezeichnung Cocamidopropyl Betaine bekannte N,N-Dimethyl-N-(lauroylamidopropyl)ammoniumacetobetain.
Die Betain-Tenside zählen zu den schäumenden Tensiden.
Bevorzugt sind vor allem solche Betain-Tenside, die in wasserfreier, feinteiliger Form verfügbar sind. Ein besonders geeignetes Produkt ist z. B. das als Tego Betain CKD im Handel erhältliche Cocamidopropyl Betaine.

Unter ampholytischen Tensiden werden solche oberflächenaktiven Verbindungen verstanden, die außer einer C₈ - C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Erfindungsgemäß bevorzugte ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈₋Acylsarcosin.

Unter nichtionischen Tensiden werden solche Tenside verstanden, die eine lipophile, bevorzugt lineare Alkyl- oder Acylgruppe mit 8-22 C-Atomen und als hydrophile Gruppe einen Glucosid- oder Polyglucosidrest, einen Glycerin- oder Polyglycerinrest, einen Sorbitanrest oder einen Polyglycoletherrest oder mehrere dieser Reste enthalten. Erfindungsgemäß bevorzugte nichtionische Tenside sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, wie beispielsweise die unter der Verkaufsbezeichnung Dehydol® LT (Cognis) erhältlichen Typen, an Fettsäuren mit 8 bis 30 C-Atomen und an Alkylphenole mit 8 bis 15 C-Atomen in der Alkylgruppe.
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter der Verkaufsbezeichnung Dehydol® LS (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxylierte Triglyceride,
- alkoxylierte Fettsäurealkylester der Formel R¹²CO-(OCH₂CHR¹³)_{w}OR¹⁴, in der R¹²CO für einen linearen oder verzweigten, gesättigten und/oder ungesättigten Acylrest mit 6 bis 22 Kohlenstoffatomen, R¹³ für Wasserstoff oder Methyl, R¹⁴ für lineare oder verzweigte Alkylreste mit 1 bis 4 Kohlenstoffatomen und w für Zahlen von 1 bis 20 steht,
- Aminoxide, z. B. N,N-Dimethyl-N-kokosalkylaminoxid,
- Amidoalkylaminoxide, z. B. Cocamidopropyldimethylaminoxid,
- Hydroxymischether, beispielsweise gemäß DE-OS 19738866,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkeriyloligoglycoside gemäß Formel R¹⁵O-[G]ₚ, in der R¹⁵ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Die Alkyl- und Alkenyloligoglycoside können sich von Aldosen bzw. Ketosen mit 5 oder 6 Kohlenstoffatomen, vorzugsweise von Glucose, ableiten. Vorzugsweise werden Alkyl- und/oder Alkenyloligoglycoside mit einem mittleren Oligomerisierungsgrad p von 1,1 bis 3,0, bevorzugt kleiner als 1,7 insbesondere 1,2 - 1,4 eingesetzt.
- Zuckertenside vom Typ der Fettsäure-N-alkylpolyhydroxyalkylamide der Formel R¹⁶CO-NR¹⁷Z, in der R¹⁶CO für einen aliphatischen Acylrest mit 6 bis 22 Kohlenstoffatomen, R¹⁷ für Wasserstoff, einen Alkyl- oder Hydroxyalkylrest mit 1 bis 4 Kohlenstoffatomen und Z für einen linearen oder verzweigten Polyhydroxyalkylrest mit 3 bis 12 Kohlenstoffatomen und 3 bis 10 Hydroxylgruppen steht.

Als nichtionische Tenside sind nur solche geeignet, die in wasserfreier Form zur Verfügung stehen. Aufgrund ihrer schäumenden und/oder hautverträglichen Eigenschaften besonders bevorzugt sind die Anlagerungsprodukte von Ethylenoxid an Fettalkohole, an Fettsäuren, an Fettsäuremono- oder diglyceride, an Fettsäurealkanolamide, an Methylglucosidfettsäureester oder an Alkyl- und Alkenyloligoglycoside sowie die Alkyl- und Alkenyloligoglycoside selbst, weiterhin Aminoxide und Amidoalkylaminoxide.

Eine weitere besonders geeignete Gruppe nichtionischer Tenside sind die Poly-(C₂-C₃)alkylen-modifizierten Silicone, deren frühere INCI-Bezeichnung Dimethicone Copolyol lautete, mit den aktuellen INCI-Bezeichnungen PEG-x Dimethicone (mit x = 2 - 20, bevorzugt 3-17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 -18, stehen), Bis-PEG/PPGc/d Dimethicone (wobei c und d unabhängig voneinander für Zahlen von 10 - 25, bevorzugt 14 - 20 und besonders bevorzugt 14 - 16, stehen) und Bis-PEG/PPGe/f PEG/PPG g/h Dimethicone (wobei e, f, g und h unabhängig voneinander für Zahlen von 10 - 20, bevorzugt 14 - 18 und besonders bevorzugt 16, stehen), die z. B. unter den Handelsbezeichnungen Dow Corning 193, Abil Care 85 oder Abil EM 97 (Goldschmidt) im Handel sind.

Erfindungsgemäß ebenfalls einsetzbar, wenn auch aufgrund der Hautverträglichkeit an der empfindlichen Gesichtshaut weniger bevorzugt, sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten der genannten Tenside weisen bevorzugt 10 bis 18 Kohlenstoffatome auf, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.
Bei Esterquats handelt es sich um bekannte Stoffe, die sowohl mindestens eine Esterfunktion als auch mindestens eine quartäre Ammoniumgruppe als Strukturelement enthalten. Erfindungsgemäß bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders bevorzugte Verbindung aus dieser Substanzgruppe stellt das unter der Bezeichnung Tegoamid® S 18 im Handel erhältliche Stearamidopropyl-dimethylamin dar.

Die erfindungsgemäßen Hautreinigungszusammensetzungen enthalten, bezogen auf die Gesamtzusammensetzung, insgesamt mindestens 1 Gew.-% mindestens eines anionischen, zwitterionischen, ampholytischen oder nichtionischen Tensids, wobei mindestens ein schäumendes Tensid enthalten ist. Bevorzugt beträgt der gesamte Tensidgehalt 5 - 30 Gew.-%, besonders bevorzugt 8 - 20 Gew.-%. Die Gesamtmenge an schäumendem Tensid beträgt in einer weiteren bevorzugten Ausführungsform der Erfindung 1 - 30 Gew.-%, besonders bevorzugt 3 - 25 Gew.-% und außerordentlich bevorzugt 5 - 15 Gew.-%.
Die meisten der vorgenannten Tenside stellen schäumende Tenside dar, das heißt, eine 1 Gew.-%ige wässrige Lösung eines dieser Tenside erzeugt nach kurzem (ca. 10 - 20 Sekunden) Schütteln Schaum, der für mindestens 15 Sekunden stabil ist. Zu den schwach oder gar nicht schäumenden Tensiden zählen beispielsweise mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, Sorbitanfettsäureester und Anlagerungsprodukte von Ethylenoxid an Sorbitanfettsäureester wie beispielsweise die Polysorbate.

Unter hydratisierenden Salzen mit negativer Lösungsenthalpie sind solche wasserlöslichen Salze zu verstehen, die sich in Wasser unter Wärmeentwicklung lösen. Dies ist in der Regel dann der Fall, wenn bei der Auflösung Hydrate gebildet werden und die Bildungswärme dieser Hydrate größer ist als die zur Überwindung der Gitterenergie verbrauchte Wärme. In der Regel handelt es sich dabei um ganz oder teilweise dehydratisierte Salze, die in Wasser Hydrate bilden. Erfindungsgemäß bevorzugte Salze dieses Typs sind z. B. Calciumchlorid, Calciumsulfat, Magnesiumchlorid, Magnesiumsulfat, Aluminiumsulfat, Chloride von Alkalimetallen, Sulfate von Alkalimetallen, z. B. Natriumchlorid oder Natriumsulfat, Carbonate und Sesquicarbonate, Ortho- und Pyrophosphate, Borate, Alkalimetallcitrate, Alkalimetallacetate, Zinkcitrat, Zinksulfat und Zinknitrat.
Erfindungsgemäß besonders bevorzugt sind die wasserlöslichen Salze, die sich in Wasser mit negativer Lösungsenthalpie lösen und nicht hydrolysieren und damit den pH-Wert des Produktes bei der Anwendung nicht beeinflussen. Die nicht-hydrolysierenden Salze sind besonders bevorzugt ausgewählt aus den wasserfreien Chloriden und Sulfaten der Alkali- und Erdalkalimetalle. Besonders bevorzugt sind Magnesiumsulfat, Natriumsulfat, Magnesiumchlorid und Calciumchlorid. Insbesondere mit diesen nicht-hydrolysierenden Salzen können pH-hautfreundliche Zusammensetzungen, die insbesondere auch zur Anwendung auf empfindlicher Haut geeignet sind, hergestellt werden.
Die erfindungsgemäßen Zusammensetzungen enthalten mindestens 5 Gew.-% mindestens eines wasserlöslichen Salzes mit negativer Lösungsenthalpie. In einer bevorzugten Ausführungsform der Erfindung ist/sind das/die wasserlösliche/n Salz/e mit negativer Lösungsenthalpie in einer Gesamtmenge von 5 - 20 Gew.-%, bevorzugt 7 - 16 Gew.-% und besonders bevorzugt 9 - 12 Gew.-%, jeweils bezogen auf die Gesamtzusammensetzung, enthalten.
Dabei ist darauf zu achten, dass Salze mit hoher Lösungswärme nicht in zu hoher Dosierung eingesetzt werden, damit es bei der Anwendung nicht zu einer unangenehmen Hitzeentwicklung auf der Haut kommt. Daher sollte z. B. wasserfreies MgSO₄ bevorzugt in Mengen von nicht mehr als 15 Gew.-% eingesetzt werden.

In einer weiteren bevorzugten Ausführungsform weisen die erfindungsgemäßen Zusammensetzungen bei Verdünnung zu einer 50 Gew.-%igen wässrigen Lösung bei 20°C einen pH-Wert im Bereich von 4,5 - 7,5, bevorzugt 4,9 - 7,0, besonders bevorzugt 4,9 - 5,5, auf.

In einer weiteren bevorzugten Ausführungsform liegen die erfindungsgemäßen Hautreinigungszusammensetzungen in Form einer gelförmigen, flüssigen bis hochviskosen Zubereitung vor. Die gelartige Konsistenz des erfindungsgemäßen Hautreinigungsmittels lässt sich durch den Einsatz geeigneter Verdickungsmittel steuern. Bevorzugt sollte die Viskosität des Produktes höher als 2 Pa.s (gemessen mit einem Brookfield Rotationsviskosimeter, Typ RTV-Helipath, Spindel 5, bei 10 UpM (Umdrehungen pro Minute) und 20°C) liegen. Für die Anwendung der erfindungsgemäßen Hautreinigungszusammensetzung aus Tuben oder Kunststoff-Spendefläschchen ist eine Viskosität von 5 - 30 Pa.s, insbesondere von 7 - 15 Pa·s, besonders bevorzugt. Weitere bevorzugte Ausführungsformen der Erfindung sind dadurch gekennzeichnet, dass sie eine Viskosität von 2 - 50 Pa·s, bevorzugt 5 - 40 Pa.s, besonders bevorzugt 8 - 30 Pa.s, aufweisen, gemessen mit einem Brookfield Rotationsviskosimeter, Typ RTV-Helipath, Spindel 5, bei 10 UpM, 20°C.

Die erfindungsgemäßen Zusammensetzungen enthalten mindestens 0,1 Gew.-% mindestens eines organischen und/oder anorganischen Verdickungsmittels.
Bevorzugte Ausführungsformen der Erfindung sind dadurch gekennzeichnet, dass sie mindestens ein organisches Verdickungsmittel enthalten, das ausgewählt ist aus den organischen Hydrokolloiden, also natürlichen oder synthetischen Polymeren, die in Wasser quellbar oder löslich sind und die sich in der im wesentlichen aus den vorgenannten Polyolen bestehenden Trägerflüssigkeit lösen oder zumindest aufquellen lassen. Besonders bevorzugte organische Verdickungsmittel sind insbesondere nichtionische Polysaccharidderivate, wie die aus α-D-Glucose-Einheiten aufgebauten Stärken, sowie Stärkeabbauprodukte wie Amylose, Amylopektin und Dextrine. Erfindungsgemäß besonders bevorzugt sind chemisch und/oder thermisch modifizierte Stärken, z. B. Hydroxypropylstärke. Weitere erfindungsgemäß bevorzugte organische Verdickungsmittel sind nichtionische Cellulose-Derivate, wie Methylcellulose, Hydroxypropylcellulose, Hydroxypropylmethylcellulose oder Hydroxyethylcellulose. Weitere erfindungsgemäß bevorzugte organische Verdickungsmittel sind Hydroxypropylguar oder synthetische Polymere wie Polyvinylpyrrolidon, Polyvinylalkohol oder Polyacrylamid. In einer besonders bevorzugten Ausführung ist in der erfindungsgemäßen Hautreinigungszusammensetzung als Verdickungsmittel mindestens ein nichtionisches Polysaccharidderivat enthalten. Das/die organische/n Verdickungsmittel ist/sind in einer Gesamtmenge von 0,01 bis 1 Gew.-%, bevorzugt 0,1 - 0,5 Gew.-%, jeweils bezogen auf die Gesamtzusammensetzung, enthalten.

Bevorzugte Ausführungsformen der Erfindung sind dadurch gekennzeichnet, dass sie mindestens ein anorganisches Verdickungsmittel enthalten, das ausgewählt ist aus Schichtsilikaten, insbesondere Tonen (Kaolinen, Montmorilloniten, Bentoniten), die organisch modifiziert sein können (z. B. Disteardimonium Hectorite oder Quatemium-18 Hectorite), und amorphen Kieselsäuren, insbesondere pyrogenen Kieselsäuren (Aerosil) oder Fällungskieselsäuren. Das/die anorganische/n Verdickungsmittel ist/sind bevorzugt in einer Gesamtmenge von 0,1 bis 5 Gew.-%, besonders bevorzugt 0,5 - 4 Gew.-%, außerordentlich bevorzugt 1 - 3 Gew.-%, jeweils bezogen auf die Gesamtzusammensetzung, enthalten.

Die erfindungsgemäßen Hautreinigungszusammensetzungen enthalten weiterhin mindestens 0,1 Gew.-% mindestens einer organischen, wasserunlöslichen und mechanisch stabilen Abrasivkomponente mit einem mittleren Teilchendurchmesser von 50 - 400 µm. Als mechanisch stabil gelten erfindungsgemäß solche Abrasivkomponenten, die beim Verreiben auf der Haut nicht mechanisch zerstört werden, wie das beispielsweise bei Wirkstoff-gefüllten Mikrokapseln erwünscht wäre. Bevorzugte Ausführungsformen der Erfindung sind dadurch gekennzeichnet, dass die organische, wasserunlösliche und mechanisch stabile Abrasivkomponente ausgewählt ist aus Polymerkügelchen, besonders bevorzugt aus Polyethylen oder Polyamid-11, weiterhin ausgewählt aus kristalliner Cellulose, gehärtetem Jojobaöl (Jojobabeads) und gemahlenen Pflanzenteilen wie Mandelkleie oder Weizenkleie sowie Mischungen dieser Komponenten. Die Abrasivkomponente weist einen mittleren Teilchendurchmesser von 50 - 400 µm, bevorzugt 75 - 350 µm, besonders bevorzugt 100 - 300 µm und außerordentlich bevorzugt 150 - 300 µm, auf.
Weitere bevorzugte Ausführungsformen der Erfindung sind dadurch gekennzeichnet, dass die organische Abrasivkomponente oder die Mischung von organischen Abrasivkomponenten in einer Menge von 0,1 bis 10 Gew.-%, bevorzugt 1-8 Gew.-% und besonders bevorzugt 2-5 Gew.-%, jeweils bezogen auf die Gesamtzusammensetzung, enthalten ist.

Darüber hinaus können in der Hautreinigungszusammensetzung auch adsorptiv wirksame, pulverförmige Stoffe enthalten sein, die sich durch eine große Oberfläche und eine mittlere Teilchengröße kleiner 0,5 µm auszeichnen. Solche Stoffe können anorganische Pulver wie z. B. Talkum, Veegum® (synthetische Magnesiumaluminiumsilikate), Zeolithe, Aluminiumoxide oder nanopartikuläre Salze sein. Es kann sich aber auch um organische, feinteilige Adsorbentien wie z. B. Cellulose, modifizierte Stärke oder Polymerpulver, wie Polyamidpulver, mit einer mittleren Teilchengröße kleiner 50 µm handeln. Ein besonders bevorzugtes teilchenförmiges Adsorptionsmittel ist Talkum. In einer bevorzugten Ausführungsform enthalten die erfindungsgemäßen Hautreinigungszusammensetzungen mindestens ein teilchenförmiges, anorganisches oder organisches Adsorptionsmittels mit einer mittleren Teilchengröße kleiner 50 µm in einer Gesamtmenge von 3 - 30 Gew.-%, bevorzugt 5 - 20 Gew.-%, besonders bevorzugt 8 - 15 Gew.-%.

Zusätzlich zu den genannten Komponenten können in dem erfindungsgemäßen Reinigungsmittel weitere in Körperreinigungsmitteln übliche Hilfsmittel und Zusätze enthalten sein. Solche Zusätze dienen der Verbesserung der Hautverträglichkeit und der kosmetischen Sensorik ganz allgemein. Geeignete Stoffe sind z. B. Feuchthaltemittel wie z. B. Pyrrolidoncarbonsäure, keratolytische und hautweichmachende Komponenten wie z. B. Harnstoff, Allantoin, rückfettende Komponenten wie z. B. emulgierte Lipidkomponenten und Silikone, dispergierte Fette und Wachse, insbesondere solche, die in mikroemulgierter oder nanopartikulärer Form vorliegen, Vitamine wie Tocopherol, Retinol oder Ascorbinsäure, Panthenol oder Biotin sowie Vitaminderivate, insbesondere Tocopherolacetat, Retinylpalmitat oder Pantolacton, wasserlösliche Proteinderivate, Duftstoffe, Farbstoffe und Perlglanzpigmente.

Weitere Hilfsmittel, die vorwiegend der Lagerstabilität dienen, sind z. B. Konservierungsstoffe, Komplexbildner, pH-Regulatoren und Puffersubstanzen.

Die Herstellung der erfindungsgemäßen Hautreinigungszusammensetzungen erfolgt durch einfaches Vermischen der Komponenten unter Erwärmen. Dabei legt man die flüssigen Trägerkomponenten, also die wasserlöslichen Polyole, vor und löst oder dispergiert darin die Verdickungsmittel, dann fügt man nacheinander die löslichen Tenside, die pulverförmigen, zu dispergierenden Tenside, die Adsorbentien und zuletzt die hydratisierenden Salze zu. Es empfiehlt sich, dabei in einer geschlossenen Anlage unter leichtem Unterdruck zu arbeiten, damit im Produkt keine Luft eingeschlossen wird und nicht zu viel Luftfeuchtigkeit zutreten kann.

Ein weiterer Gegenstand der vorliegenden Erfindung ist die Verwendung einer erfindungsgemäßen Hautreinigungszusammensetzung gemäß einem der Patentansprüche 1 - 9 zur Make-up-Entfernung und/oder als Duschgel.

Ein weiterer Gegenstand der vorliegenden Erfindung ist die Verwendung einer erfindungsgemäßen Hautreinigungszusammensetzung gemäß einem der Patentansprüche 1 - 8 zur Ausrüstung von flexiblen, wasserunlöslichen Trägern, insbesondere ein- oder mehrlagigen Tüchern aus Geweben oder nicht-gewebten Vliesen sowie Schwämmen.
Geeignete und bevorzugte Verpackungsbehältnisse für die erfindungsgemäße Hautreinigungszusammensetzung können vorteilhaft aus einer Tube oder aus einer Kunststoffflasche mit einem Pumpspenderaufsatz bestehen. Vorteilhafterweise kann die erfindungsgemäße Hautreinigungszusammensetzung auch mit einem Treibmittel aufgeschäumt werden und in einer Aerosoldose verpackt sein.

Ein weiterer Gegenstand der vorliegenden Erfindung ist eine Hautreinigungszusammensetzung, enthaltend
(i) eine kosmetische und/oder dermatologische Zubereitung A in einer Menge von 10 bis 90 Gewichts-% der Gesamtzubereitung, die Wasser enthalten kann,
(ii) eine Zubereitung B in einer Menge von 90 bis 10 Gewichts-% der Gesamtzubereitung, enthaltend (jeweils bezogen auf die Zusammensetzung B)
   a) mindestens 30 Gew.-% mindestens eines wasserlöslichen Polyols, ausgewählt aus wasserlöslichen mehrwertigen C₂ - C₉-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten,
   b) mindestens 1 Gew.-% mindestens eines anionischen, zwitterionischen, ampholytischen oder nichtionischen Tensids oder Mischungen hiervon, wobei mindestens ein schäumendes Tensid enthalten ist,
   c) mindestens 5 Gew.-% mindestens eines wasserlöslichen Salzes, das sich in Wasser mit negativer Lösungsenthalpie löst,
   d) mindestens 0,1 Gew.-% mindestens eines organischen und/oder anorganischen Verdickungsmittels sowie
   e) mindestens 0,1 Gew.-% mindestens einer organischen, wasserunlöslichen und mechanisch stabilen Abrasivkomponente mit einem mittleren Teilchendurchmesser von 50 - 400 µm,

dadurch gekennzeichnet, dass die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

Als erfindungsgemäß bevorzugte Verpackungsbehältnisse können beispielsweise Verpackungen gewählt werden, wie sie in der WO 96/37420 oder der GB 2 180 215 offenbart sind. Auch Verpackungssysteme, wie sie von mehrfarbig gestreifter Zahnpasta her bekannt sind, sind erfindungsgemäß bevorzugt mit der Maßgabe, dass die beiden Zubereitungen durch eine undurchlässige Sperre voneinander getrennt sind und erst beim Austritt aus der Verpackung zu einer streifenförmigen Gesamtzusammensetzung zusammengesetzt werden.

Weitere erfindungsgemäß bevorzugte Verpackungsbehältnisse stellen ferner mit zwei Kammern versehene Quetschflaschen dar, die entweder für beide Zubereitungen eine gemeinsame Entnahmeöffnung oder aber zwei separate Entnahmeöffnungen aufweisen.

Weitere erfindungsgemäß bevorzugte Verpackungsbehältnisse stellen auch Doppelkammertuben mit ein oder zwei Entnahmeöffnungen dar.

Weiterhin ist es erfindungsgemäß bevorzugt, wenn beide Zubereitungen zusammen oder getrennt mit Hilfe eines Pump- und/oder Dosierspenders dem Verpackungsbehältnis entnommen werden können.

Besonders bevorzugt sind Verpackungsbehältnisse, bei denen die beiden Zubereitungen A und B dem Behältnis aus zwei unterschiedlichen Entnahmeöffnungen entnommen werden.

Erfindungsgemäß besonders bevorzugte Ausführungsformen sind dadurch gekennzeichnet, dass die beiden Zubereitungen A und B unterschiedlich gefärbt und/oder mit unterschiedlichen Wirkstoffen beladen sind.

Die folgenden Beispiele sollen den Gegenstand der Erfindung näher erläutern, ohne ihn hierauf zu beschränken.

Es wurden folgende Beispielzusammensetzungen hergestellt
(Angaben in Gew.-%):

| | Nr.1 | Nr. 2 | Nr. 3 | Nr. 4 | Nr. 5 | Nr. 6 |
|---|---|---|---|---|---|---|
| 1,2-Propylenglycol | 8 | 25 | 30 | 25 | 25 | 13 |
| 1,3-Butylenglycol | 12 | - | - | 5 | - | - |
| Polyethylenglycol 400 | 25 | 20 | 20 | 15 | 25 | 30 |
| Sorbit (100 %, Pulver) | 1 | 10 | 10 | 5,0 | 10 | 3,0 |
| Glycerin (99,5 %ig) | 1 | 2,0 | 2,0 | 2,0 | - | 5,0 |
| Dow Corning® 193 | 3 | 1,0 | - | 1,0 | 1,0 | - |
| Polysorbate 20 | 1,5 | 1,0 | 1,5 | - | 2,0 | 1,5 |
| Elfan® AT 84 | 6 | - | 10 | 20 | - | 8,0 |
| Tego Betain® CKD | 3 | 15 | 10 | - | 15 | - |
| Sulfosuccinat 128 P | 10 | - | 3,0 | - | 5,0 | 5,0 |
| Parfümöl | 0,5 | 0,3 | 0,3 | 0,3 | 0,3 | 0,5 |
| Klucel® M | - | 0,2 | 0,2 | 0,3 | 0,3 | - |
| Talkum | 17 | 11,0 | - | 16,4 | 4,4 | 12,0 |
| Neosil® CT 11 | 2 | 0,5 | - | - | 1,0 | - |
| Aerosil® 200 | - | - | 2,0 | 1,0 | - | 4,0 |
| Na₂SO₄ | - | - | - | - | - | - |
| MgSO₄ | 9 | 12 | 10,0 | 8,0 | 10 | 13 |
| Microscrub 50 PC | 1 | 2 | 1 | 1 | 1 | 5 |
| Viskosität (Pa·s) (Brookfield, Type RTV, Spindel 5, 10 UpM, 20°C) | 7,2 | - | - | - | - | - |
| Viskosität (Pa·s) (Brookfield, Type RTV, Spindel D, 20 UpM, 20°C) | - | 4 | 11 | 29 | 9 | |
| Viskosität (Pa·s) (Brookfield, Type RTV, Spindel TC, 4 UpM, 20°C) | - | - | - | - | - | 20-25 |
| pH-Wert in 50 Gew.-%iger | 5,1 | - | - | - | - | - |
| wässriger Lösung (20°C) | 5,4 | | | | | |

### Es wurden folgende Handelsprodukte verwendet:

| | |
|---|---|
| Cetiol® HE (Cognis Deutschland): | Glycerin + 7,3 EO-Kokosfettsäureester |
| Elfan® AT 84 (Akzo Nobel): | Kokosacylisethionat, Na-Salz (Pulver mit 84 Gew.-% Aktivsubstanz) |
| Tego Betain® CKD (Goldschmidt): | Kokosacylamidopropyl-Betain (Pulver 82 Gew.-% Aktivsubstanz) |
| Sulfosuccinat 128 P (Cognis): | Sulfobernsteinsäure-mono(C₁₂₋₁₈)-alkylester, Di-Na-Salz (Pulver, 90 % Aktivsubstanz) |
| Klucel® M (Hercules): | Hydroxypropylcellulose (Pulver) |
| Neosil® CT11 (Crossfield) | Kieselsäure, amorph |
| Aerosil® 200 (Degussa): | Kieselsäure, pyrogen, amorph |
| Polyethylenglycol 400 | PEG-8 |
| Dow Corning® 193: | PEG-12 Dimethicone |
| Microscrub 50 PC (Micro Powders Inc.) | Polyethylene |

## Patentansprüche

1. Wasserfreie, selbsterwärmende Hautreinigungszusammensetzung, die beim Vermischen mit Wasser Hydratationswärme erzeugt, enthaltend (jeweils bezogen auf die Gesamtzusammensetzung):
a) mindestens 30 Gew.-% mindestens eines wasserlöslichen Polyols, ausgewählt aus wasserlöslichen mehrwertigen C₂ - C₉-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten,
b) mindestens 1 Gew.-% mindestens eines anionischen, zwitterionischen, ampholytischen oder nichtionischen Tensids oder Mischungen hiervon, wobei mindestens ein schäumendes Tensid enthalten ist,
c) mindestens 5 Gew.-% mindestens eines wasserlöslichen Salzes, das sich in Wasser mit negativer Lösungsenthalpie löst,
d) mindestens 0,1 Gew.-% mindestens eines organischen und/oder anorganischen Verdickungsmittels sowie
e) mindestens 0,1 Gew.-% mindestens einer organischen, wasserunlöslichen und mechanisch stabilen Abrasivkomponente mit einem mittleren Teilchendurchmesser von 50 - 400 µm.

2. Hautreinigungszusammensetzung gemäß Anspruch 1, **dadurch gekennzeichnet, dass** das/die wasserlösliche/n Salz/e, das/die sich in Wasser mit negativer Lösungsenthalpie löst/lösen, nicht hydrolysiert/en.

3. Hautreinigungszusammensetzung gemäß Anspruch 2, **dadurch gekennzeichnet, dass** das/die wasserlösliche/n Salz/e, das/die sich in Wasser mit negativer Lösungsenthalpie löst/lösen und nicht hydrolysiert/en, ausgewählt ist/sind aus den wasserfreien Chloriden und Sulfaten der Alkali- und Erdalkalimetalle.

4. Hautreinigungszusammensetzung gemäß Anspruch 1 oder 2, **dadurch gekennzeichnet, dass** das/die wasserlösliche/n Salz/e in einer Gesamtmenge von 5 - 20 Gew.-%, bevorzugt 7 - 16 Gew.-% und besonders bevorzugt 9 - 12 Gew.-%, jeweils bezogen auf die Gesamtzusammensetzung, enthalten ist/sind.

5. Hautreinigungszusammensetzung gemäß einem der vorstehenden Ansprüche, **dadurch gekennzeichnet, dass** das/die wasserlösliche/n Polyol/e (Komponente a) in einer Gesamtmenge von 30 - 70 Gew.-%, bevorzugt 40 - 60 Gew.-% und besonders bevorzugt 45 - 55 Gew.-%, bezogen auf die Gesamtzusammensetzung, enthalten ist/sind.

6. Hautreinigungszusammensetzung gemäß einem der vorstehenden Ansprüche, **dadurch gekennzeichnet, dass** das schäumende Tensid ausgewählt ist aus Alkylpolyglycolethersulfaten, Acylisethionaten, Acyltauriden, Acylsarkosiden, Sulfobernsteinsäuremonoalkylester-Salzen, Alkylsulfaten, Alkansulfonaten, Alpha-Olefinsulfonaten und Alkylpolyglycolethercarboxylaten in Form ihrer Alkali-, Magnesium-, Ammonium- oder Alkanolammoniumsalze sowie aus Betain-Tensiden.

7. Hautreinigungszusammensetzung gemäß einem der vorstehenden Ansprüche, **dadurch gekennzeichnet, dass** die Zusammensetzung in einer 50 Gew.-%igen wässrigen Lösung bei 20°C einen pH-Wert im Bereich von 4,5 - 7,5, bevorzugt 4,9 - 7,0, besonders bevorzugt 4,9 - 5,5, aufweist.

8. Hautreinigungszusammensetzung gemäß einem der vorstehenden Ansprüche, **gekennzeichnet durch** eine Viskosität von 2 - 50 Pa·s, bevorzugt 5 - 40 Pa·s, besonders bevorzugt 8 - 30 Pa.s, gemessen mit einem Brookfield Rotationsviskosimeter, Typ RTV-Helipath, Spindel 5, bei 10 UpM, 20°C.

9. Hautreinigungszusammensetzung gemäß einem der vorstehenden Ansprüche, **dadurch gekennzeichnet, dass** die organische, wasserunlösliche und mechanisch stabile Abrasivkomponente mit einem mittleren Teilchendurchmesser von 50 - 400 µm ausgewählt ist aus Polymerkügelchen, bevorzugt aus Polyethylen oder Polyamid-11, kristalliner Cellulose, gehärtetem Jojobaöl (Jojobabeads) und gemahlenen Pflanzenteilen wie Mandelkleie oder Weizenkleie sowie Mischungen dieser Komponenten.

10. Verwendung einer Hautreinigungszusammensetzung gemäß einem der Ansprüche 1 - 9 zur Make-up-Entfernung und/oder als Duschgel.

11. Verwendung einer Hautreinigungszusammensetzung gemäß einem der Ansprüche 1 - 8 zur Ausrüstung flexibler, wasserunlöslicher Träger, insbesondere ein- oder mehrlagiger Tücher aus Geweben oder nicht-gewebten Vliesen sowie Schwämmen.

12. Hautreinigungszusammensetzung, enthaltend
(1) eine kosmetische und/oder dermatologische Zubereitung A in einer Menge von 10 bis 90 Gewichts-% der Gesamtzubereitung, die Wasser enthalten kann,
(2) eine Zubereitung B in einer Menge von 90 bis 10 Gewichts-% der Gesamtzubereitung, enthaltend (jeweils bezogen auf die Zusammensetzung B)
a) mindestens 30 Gew.-% mindestens eines wasserlöslichen Polyols, ausgewählt aus wasserlöslichen mehrwertigen C₂ - C₉-Alkanolen mit 2 - 6 Hydroxylgruppen, wasserlöslichen Polyethylenglycolen mit 3 - 20 Ethylenoxid-Einheiten, wasserlöslichen Polypropylenglycolen und wasserlöslichen Anlagerungsprodukten von Ethylenoxid an Propylenglycol oder an Polypropylenglycol sowie Mischungen dieser Komponenten,
b) mindestens 1 Gew.-% mindestens eines anionischen, zwitterionischen, ampholytischen oder nichtionischen Tensids oder Mischungen hiervon, wobei mindestens ein schäumendes Tensid enthalten ist,
c) mindestens 5 Gew.-% mindestens eines wasserlöslichen Salzes, das sich in Wasser mit negativer Lösungsenthalpie löst,
d) mindestens 0,1 Gew.-% mindestens eines organischen und/oder anorganischen Verdickungsmittels sowie
e) mindestens 0,1 Gew.-% mindestens einer organischen, wasserunlöslichen und mechanisch stabilen Abrasivkomponente mit einem mittleren Teilchendurchmesser von 50 - 400 µm,
**dadurch gekennzeichnet, dass** die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.
